# EUROPEAN PATENT APPLICATION

(11) **EP 2 559 436 A1**
(43) Date of publication of application: **20.02.2013**
(21) Application number: 11768496.9
(22) Date of filing: 15.04.2011
(51) Int. Cl.: A61K 33/38, C01B 33/26, B82B 1/00, B82Y 5/00

(54) **COMPOSITION COMPRISING ALUMINUM SILICATES AND SILVER NANOPARTICLES AS BACTERICIDES**

(30) Priority: 16.04.2010 ES 201030557
(71) Applicant: CONSEJO SUPERIOR DE INVESTIGACIONES CIENTIFICAS (CSIC), 28006 Madrid (ES)
(72) Inventor: CABAL ÁLVAREZ, María Belen, E-28049 Madrid (ES); MOYA CORRAL, José S., E-28049 Madrid (ES)
(74) Representative: Pons Ariño, Angel
(86) International application number: PCT/ES2011/070272
(87) International publication number: WO 2011/128488

(57) **Abstract**

The present invention relates to a composition of a nanocomposite or nanostructured powder comprising an aluminum silicate and silver nanoparticles measuring less than 50 nm distributed over the surface thereof. The invention also relates to the use of said composition as a bactericide and to a production method thereof.

## Description

The present invention relates to a composition comprising aluminium silicate and silver nanoparticles with a size of less than 50 nm distributed on the surface thereof, to its use as a bactericide and to a process for obtaining said composition.

### PRIOR STATE OF THE ART

It is well-known that silver at low concentrations has antibacterial properties against a wide range of pathogens, including the common bacterial strains that cause implant-associated infections, and is non-toxic for mammalian cells. Most biomaterials that contain silver as an antimicrobial substance are composed of the elementary or cationic form of the metal supported by both organic and inorganic matrices. The antimicrobial activity has been studied in the case of polymers and bioglasses that contain silver, but not in the case of nanostructured aluminium silicate-silver composite materials.

Recently, studies have been published on the obtainment of composites of kaolin with silver nanoparticles [Patakfalvi, P., Dékány, I. Applied Clay Science, 2004, 25, pp. 149 - 159; Patakfalvi, P., Dékány, I. Proceeding of SPIE-The international society for optical engineering, 2003, 5118, pp. 657 - 667; Patakfalvi, P., Oszkó, A., Dékány, I. Colloids and Surface A: Physicochemical and Engineering Aspects, 2003, 220, pp. 45 - 54]. These works propose the synthesis of silver nanoparticles in the interlaminar space of kaolin, the latter having been previously disaggregated by interspersing dimethyl sulfoxide. In none of the cases cited was the antimicrobial capacity of these materials evaluated; they were only presented as potential applications for uses such as photosensitive components, catalysts, in photocatalysis, surface-enhanced Raman scattering spectroscopy and chemical analysis.

The biocidal activity of silver nanoparticles is influenced by their size: the smaller the size, the greater the antimicrobial activity, for which reason the agglomeration of nanoparticles is a problem for said activity.

### DESCRIPTION OF THE INVENTION

The inventors of the present application have found a solution to prevent the agglomeration of silver nanoparticles by using said nanoparticles on the surface of different substrates, in particular aluminium silicates, which confer upon said composition the characteristic of zero toxicity and makes it possible for it to be used in medical and textile applications, amongst others.

The present invention provides a nanocomposite or nanostructured powder composition that comprises an aluminium silicate and, adhered onto the surface thereof, silver nanoparticles with sizes of less than 50 nm. It also provides a process for obtaining said composition, as well as the use thereof as a bactericide.

Therefore, a first aspect of the present invention relates to a composition (hereinafter composition of the invention) that comprises an aluminium silicate and silver nanoparticles with a size of less than 50 nm distributed on the surface thereof. Preferably, the size of the silver nanoparticles is less than 20 nm.

In a preferred embodiment, said silver nanoparticles are deposited on the surface of the aluminium silicate, and said aluminium silicate may be both hydrated and non-hydrated.

Preferably, the aluminium silicate is selected from kaolin, metakaolin, montmorillonite, mica or any combination thereof. More preferably, the aluminium silicate is kaolin.

In a preferred embodiment, the percentage of silver ranges between 0.01 % and 15 % by weight with respect to the final composition. In a more preferred embodiment, the percentage of silver ranges between 0.1 % and 0.8 % by weight.

A second aspect of the present invention relates to a process for obtaining the composition of the invention (hereinafter process of the invention), which comprises the following steps:
a) aqueous suspension of the aluminium silicate with a surfactant agent,
b) addition of a silver precursor to the suspension obtained in (a), and
c) reduction of the silver in the product obtained in (b).

In the present invention, "surfactant agent" is understood to mean that substance or product which has the capacity to reduce the surface tension between two surfaces in contact with one another. Surfactant agents are molecules that have a hydrophilic part and a hydrophobic part, and are known to any person skilled in the art.

In a preferred embodiment, the process of the invention further comprises adjusting the pH of the suspension obtained in step (a) to between 6 and 7.

In a preferred embodiment, the process of the invention further comprises a step (b') of precipitation of silver ions at a pH of between 8 and 10 by stirring the suspension obtained in step (b).

Preferably, the process of the invention further comprises a filtration, washing with water and drying of the product obtained in step (b') at temperatures ranging between 50 °C and 100 °C.

In another preferred embodiment, the process of the invention further comprises a filtration, washing with water and drying of the product obtained in step (c) at temperatures ranging between 50 °C and 100 °C.

Preferably, the silver precursor is AgNO₃.

In a preferred embodiment, the reduction is performed with a reducing agent that is selected from H₂ and NaBH₄.

The main advantage of the process of the invention with respect to the current state of the art is the fact that it prevents the agglomeration of the silver nanoparticles, since these are adhered to the substrate, i.e. the aluminium silicate.

In an alternative to the process of the invention, the latter is performed by depositing the silver nanoparticles on the surface of the aluminium silicate (hydrated or non-hydrated). Thus, the process comprises the following steps:
a) Preparation of an aqueous suspension of aluminium silicate whereto a low concentration of surfactant, preferably an anionic surfactant, is added,
b) adjusting the pH to between 6 and 7 with an aqueous solution of 1M HNO₃, such that a good dispersion of kaolin is maintained, whilst the pH conditions are far from favouring the precipitation of the silver precursor,
c) addition, in the absence of light, of an aqueous solution of the silver precursor salt with the necessary concentration for the elementary silver content to range between 0.01 % and 8 % weight percent in the final composite, with respect to the aluminium silicate solids content, preferably 1 % weight percent of silver,
d) strong stirring of the suspension, adjusting the pH to 9, such that the Ag⁺ cations are precipitated as an oxide, Ag₂O,
e) filtration, washing with distilled water and drying of the resulting powder, and
f) reduction in an H₂ atmosphere within the temperature range 150 °C - 500 °C, preferably 350 °C.

Another alternative to the process of the invention relates to the deposition of silver, which comprises the following steps:
a) Preparation of an aqueous suspension with the aluminium silicate powder whereto a low concentration of surfactant, preferably an anionic surfactant, is added,
b) Adjusting the pH to between 6 and 7, with an aqueous solution of 1 M HNO₃, such that a good dispersion of the aluminium silicate is maintained, whilst the pH conditions are far from favouring the precipitation of the silver precursor,
c) Drop-by-drop addition of the necessary quantity of the solution of the silver precursor, preferably AgNO₃, to obtain a final product with a concentration of Ag⁰ ranging between 0.01 % and 8 % weight percent in the final composite, maintaining strong stirring for 10 minutes, preferably 1 % weight percent of silver,
d) Chemical reduction of the silver in situ, using any radiation or reducing chemical agent, preferably NaBH₄, which is added drop-by-drop to the dispersion, whilst maintaining the strong stirring, and
e) Filtration, washing with distilled water and drying in an oven at 60 °C.

A third aspect of the present invention relates to the use of the composition as previously described as a high-efficacy bactericide, as shown in the examples of the invention.

In the present invention, "bactericide" is understood to mean those substances used for the destruction of bacteria.

The composition of the invention may be applied in the sector of surgical implants, public-use facilities (healthcare, hospitals, transport, etc.), air-conditioning equipment, foodstuffs, dental sector, paints, clothing garments, packaging (food, domestic elements, pharmaceuticals, medical devices).

Another advantage of the composition of the invention as a bactericide is the low toxicity that it presents, which is demonstrated upon verifying that the material lixiviates a quantity of silver of less than 3 ppm, and these levels are well below the toxic level.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following examples and drawings are provided for illustrative purposes, and are not intended to limit the scope of the present invention.

### DESCRIPTION OF THE FIGURES

Figure 1. - Shows the micrography obtained by Transmission Electronic Microscopy, where we may observe the homogeneous distribution of silver nanoparticles smaller than 20 nm adhered to the kaolin surface, approximately, obtained by means of Method 1.
Figure 2. - Shows the micrography obtained by Transmission Electronic Microscopy, where we may observe a nanocomposite powder obtained by means of Method 2, and it may also be observed that the Ag nanoparticles are smaller than 20 nm.

### EXAMPLES

Below we will illustrate the invention by means of assays performed by the inventors, which show the specificity and effectiveness of the composition comprising aluminium silicate with silver nanoparticles as a bactericide and the process for obtaining it.

### Example 1. - Obtainment of the nanocomposite powders of the invention. Process for depositing silver on the kaolin or metakaolin nanoparticles

We describe the process for depositing silver nanoparticles on the surface of the aluminium silicate (hydrated or non-hydrated), in order to obtain the nanocomposite powder of the invention, which is explained below.

From this point on, the nanostructured powders of the invention were obtained by means of two methods.

### Method 1.

Starting from a precursor (for example, silver nitrate), the silver oxide is deposited on an aqueous dispersion of kaolin with the optimal quantity of surfactant. Subsequently, the Ag⁺ cation is reduced to Ag⁰ in an H₂ atmosphere in an oven, as explained below:
a) An aqueous suspension of kaolin or metakaolin is prepared. In order to achieve a better dispersion of the kaolin or metakaolin, an anionic surfactant at a low concentration is introduced as a dispersant (1 % by weight with respect to the solids concentration of kaolin or metakaolin);
b) The pH is adjusted to 6.5 with an aqueous solution of 1 M HNO₃, such that a good dispersion of the kaolin is maintained, whilst the pH conditions are far from favouring the precipitation of the silver precursor;
c) Protected from light, an aqueous solution of the silver precursor salt is added, with the concentration necessary for the elementary silver content to range between 0.01 % and 15 % weight percent in the final kaolin or metakaolin-Ag composite (with respect to the solids content of kaolin or metakaolin);
d) Whilst strongly stirring the suspension, the pH is adjusted to 9, so that the Ag⁺ cations are precipitated as an oxide, Ag₂O, and
e) Following filtration and washing, it is dried and reduced in an H₂ atmosphere within a temperature range of 150 °C - 500 °C.

The process with metakaolin was performed in the same manner.

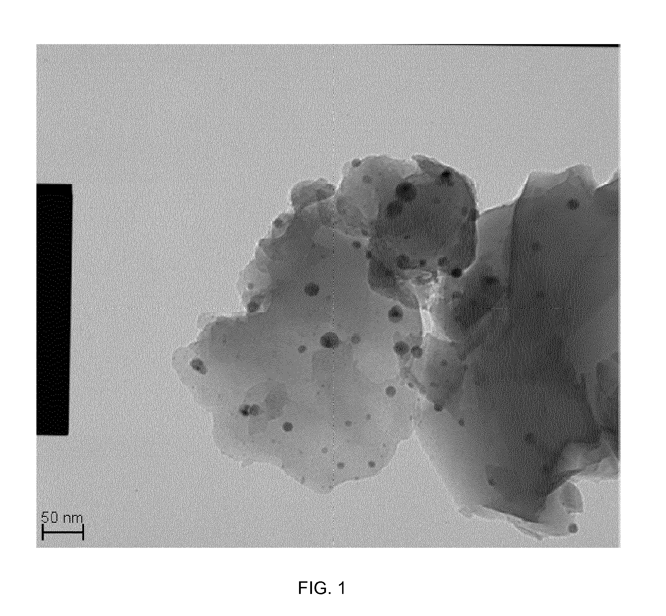

In this way, we obtained a nanocomposite powder with silver nanoparticles smaller than 20 nm adhered to the surface of a kaolin or metakaolin nanoparticle, approximately, with a homogeneous distribution, as may be observed in Figure 1.

### Method 2.

Silver nanoparticles, Ag⁰, are deposited on kaolin or from a silver precursor dispersed in water under optimal pH and dispersing conditions. The reduction is performed in situ by means of radiation or a reducing agent at room temperature, as explained below:
a) An aqueous suspension with the kaolin or metakaolin powder is prepared. In order to achieve a better dispersion of the kaolin or metakaolin, an anionic surfactant at a low concentration is introduced as a dispersant (Dolapix);
b) the pH is adjusted to 6.5 with an aqueous solution of 1 M HNO₃ in order to achieve a good dispersion of the kaolin particles, whilst preventing the precipitation of the Ag⁺ ions as Ag₂O;
c) in order to obtain, in the final product, an Ag⁰ concentration ranging between 0.01 % and 15 % weight percent in the final kaolin or metakaolin-Ag composite, the necessary quantity of the precursor, AgNO₃, is added. Once it has been added drop-by-drop to the kaolin or metakaolin dispersion, it is strongly stirred for 10 min before proceeding to the following step. It is necessary to perform this process whilst protecting the solution with the precursor and the dispersion from light once the precursor has been added;
d) the reduction of silver is performed chemically in situ, using, for example, NaBH₄, as a reducing agent, which reacts with the silver at a 1: 8 (NaBH₄: Ag⁺) molar ratio, according to the reactions:

   8(Ag⁺ + 1e⁻ ⇄ Ag⁰)

   BH⁻₄ + 3H₂O ⇄ B(OH)₃ + 7H ⁺ + 8e⁻

   8Ag⁺ + BH₄ + 3H₂O ⇄ Ag⁰ + B(OH)₃ + 7H⁺
e) the NaBH₄ solution is deposited drop-by-drop on the dispersion; and
f) it is strongly stirred, filtered, washed with distilled water and, finally, dried in an oven at 60 °C.

The process with metakaolin was performed in the same manner.

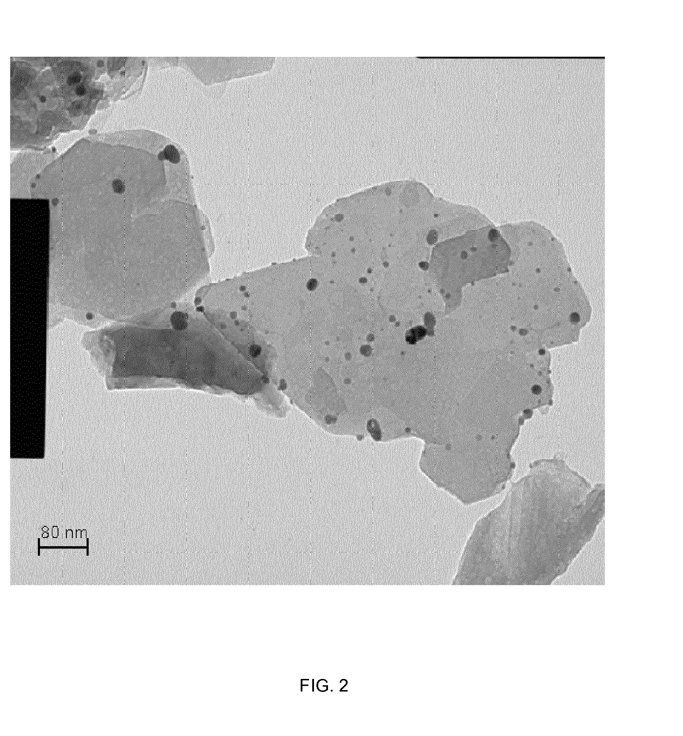

Thus, we obtained a nanocomposite powder of the invention, where it may be observed that the Ag nanoparticles are smaller than 20 nm, as may be observed in Figure 2.

### Example 2. - Biocidal activity and leaching tests for the nanocomposite powders of the invention

Bactericidal tests were performed in order to investigate the effect of the samples containing silver on different organisms: Escherichia coli JM 110 (Gram-negative bacteria), Micrococcus luteus (Gram-positive bacteria). The microorganisms were seeded in a solid medium, Petri dishes, from Luria Bertani (LB) (containing: 1 % tryptone, 0.5 % yeast extract, 1 % NaCl, 1.5 % agar). The dishes were incubated for 24 hours at 37 °C. Subsequently, colonies of each microorganism isolated from the aforementioned dishes were inoculated in 1 ml of LB and cultured at 37 °C for 5 hours in order to obtain the precultures. Parallel to this, suspensions of 300 mg/ml (weight/weight) in water of the preparations according to Method 2, containing 1 % silver, were prepared. Finally, 10 µl of each of the microorganism precultures were inoculated in 1 ml of LB. 150 µl of the kaolin-nAg samples were added to the cultures, leading to a final concentration of 0.036 % weight percent of Ag. Moreover, as a control, samples without silver were prepared, composed of a mixture of water plus the nutrient. The cultures were incubated at 37 °C under stirring and aliquots of the different cultures were collected for the viable count following serial dilutions of the different cultures.

### 2.1. - Biocidal test performed with Micrococcus luteus

An aqueous suspension (9 % weight percent of solids) was prepared with the kaolin powder obtained using Method 2 (AgNO₃ was used as the silver precursor), and the silver content in the final composite was 1 % weight percent (with respect to the solids content of kaolin). The test performed with Micrococcus luteus shows a titre at 24 hours of < 1.0 × 10⁴, whereas the control is 1.0 × 10¹².

After 72 hours, the concentration of silver lixiviated in the culture medium was < 3 ppm.

### 2.2. - Biocidal test performed with Escherichia coli

An aqueous suspension (9 % weight percent of solids) was prepared with the kaolin powder obtained using Method 2 (AgNO₃ was used as the silver precursor), and the silver content in the final composite was 1 % weight percent (with respect to the solids content of kaolin). The test performed with Escherichia coli JM 110 shows a titre at 24 hours of < 1.0 × 10⁴, whereas the control is 2.93 × 10¹².

After 72 hours, the concentration of lixiviated silver in the culture medium was < 3 ppm.

## Claims

1. Composition that comprises an aluminium silicate with silver nanoparticles with a size of less than 50 nm distributed on the surface thereof.

2. Composition according to claim 1, where the size of the silver nanoparticles is less than 20 nm.

3. Composition according to any of claims 1 or 2, where the aluminium silicate is selected from kaolin, metakaolin, montmorillonite, mica or any combination thereof.

4. Composition according to claim 3, where the aluminium silicate is kaolin.

5. Composition according to any of claims 1 to 4, where the weight percentage of silver ranges between 0.01 % and 15 %.

6. Composition according to claim 5, where the weight percentage of silver ranges between 0.1 % and 0.8 %.

7. Process for obtaining the composition according to any of claims 1 to 6, which comprises the following steps:
a. aqueous suspension of the aluminium silicate with a surfactant agent,
b. addition of a silver precursor to the suspension obtained in (a), and
c. reduction of the silver in the product obtained in (b).

8. Process according to claim 7, which further comprises adjusting the pH of the suspension obtained in step (a) to between 6 and 7.

9. Process according to any of claims 7 or 8, which further comprises a step (b') of precipitation of silver ions at a pH of between 8 and 10 by stirring the suspension obtained in step (b).

10. Process according to any of claims 7 to 9, which further comprises a filtration, washing with water and drying of the product obtained in step (b') at temperatures ranging between 50 °C and 100 °C.

11. Process according to any of claims 7 to 10, which further comprises a filtration, washing with water and drying of the product obtained in step (c) at temperatures ranging between 50 °C and 100 °C.

12. Process according to any of claims 7 to 11, where the silver precursor is AgNO₃.

13. Process according to any of claims 7 to 12, where the reduction is performed using a reducing agent selected from H₂ or NaBH₄.

14. Use of the composition according to any of claims 1 to 6 as a bactericide.
